# EUROPÄISCHE PATENTSCHRIFT

(11) **EP 0 759 914 B1**
(45) Veröffentlichungstag und Bekanntmachung des Hinweises auf die Patenterteilung: **23.09.1998**
(21) Anmeldenummer: 95920018.9
(22) Anmeldetag: 10.05.1995
(51) Int. Cl.: C07D 295/02, C08G 18/20

(54) **TERTIÄRES DIAMIN, VERWENDUNG ALS KATALYSATOR UND VERFAHREN ZUR HERSTELLUNG VON TERTIÄREN DIAMINEN**
TERTIARY DIAMINE, ITS USE AS CATALYST AND METHOD FOR PREPARATION OF TERTIARY DIAMINES
DIAMINE TERTIAIRE, SON UTILISATION COMME CATALYSEUR, ET PROCEDE DE PREPARATION DE DIAMINES TERTIAIRES

(30) Priorität: 10.05.1994 CH 1460/94
(43) Veröffentlichungstag der Anmeldung: 05.03.1997
(73) Patentinhaber: LONZA AG, CH-3945 Gampel/Wallis (CH)
(72) Erfinder: HEVELING, Josef, CH-3904 Naters (CH); GERHARD, Andreas, CH-3930 Visp (CH); DAUM, Ulrich, CH-4114 Hofstetten (CH)
(74) Vertreter: Weinhold, Peter, Dr.
(86) Internationale Anmeldenummer: EP9501773
(87) Internationale Veröffentlichungsnummer: WO9530666

(56) Entgegenhaltungen:
- EP-A- 0 415 263
- DE-B- 1 032 920
- DE-B- 1 034 180
- US-A- 3 787 496

## Beschreibung

Die vorliegende Erfindung betrifft ein Verfahren zur Herstellung von tertiären Diaminen mit zwei durch eine aliphatische Kohlenstoffkette verbundenen gesättigten heterocyclischen Ringen aus aliphatischen Dinitrilen sowie ein neues tertiäres Diamin und dessen Verwendung als Katalysator zur Herstellung von Polyurethanen.

Tertiäre Diamine wie beispielsweise das 1,5-Dipiperidinopentan sind wegen ihrer hohen Basizität und relativ geringen Flüchtigkeit wertvolle Verbindungen, die beispielsweise als Härter in Epoxidharzen eingesetzt werden können (DE-PS 10 32 920).
Eine neuere Verwendung derartiger Verbindungen ist der Einsatz als Ausgangsmaterial zur Herstellung von Emulsionsstabilisatoren für Blutersatzstoffe (EP-A 0 415 263).

Die bisher bekannten Herstellungsverfahren für diese Verbindungen (s. z. B. EP-A 0 415 263, S. 8) erfordern in der Regel zwei verschiedene Ausgangssubstanzen und liefern teils unerwünschte Abfallprodukte (Salze), teils unbefriedigende Ausbeuten. Es sind auch Verfahren bekannt, bei denen beispielsweise Piperidin unter Einfluss eines Katalysators zu 1,5-Dipiperidinopentan umgesetzt wird (DE-PS 10 34 180). Auch diese Verfahren erbringen oft nur schlechte Ausbeuten.

Aufgabe der vorliegenden Erfindung war daher, ein einfaches Verfahren zur Verfügung zu stellen, das tertiäre Diamine mit zwei durch eine aliphatische Kohlenstoffkette verbundenen gesättigten heterocyclischen Ringen in guter Ausbeute und ohne Anfall grösserer Mengen von Abfallprodukten liefert.

Erfindungsgemäss wird diese Aufgabe durch das Verfahren nach Patentanspruch 1 gelöst.

Es wurde überraschend gefunden, dass tertiäre Diamine der allgemeinen Formel 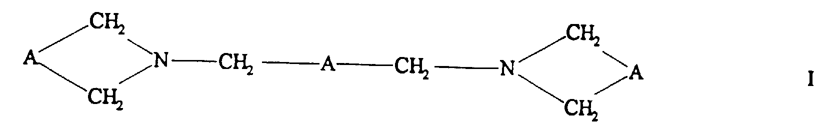 worin A jeweils gleich ist und für eine zwei- bis viergliedrige aliphatische Kohlenstoffkette steht, welche gegebenenfalls mit einer oder mehreren C₁-C₄-Alkylgruppen substituiert sein kann, in einer Stufe durch Hydrierung der entsprechenden aliphatischen Dinitrile der allgemeinen Formel

N≡C-A-C≡N II

worin A die oben genannte Bedeutung hat, hergestellt werden können. Als Nebenprodukt werden hierbei auf 1 mol Diamin 4 mol Ammoniak gebildet.

Die Hydrierung wird zweckmässig bei einer Temperatur von 100 - 250 °C und unter erhöhtem Druck in Gegenwart eines Palladium-Trägerkatalysators durchgeführt.

Als Palladium-Trägerkatalysator wird vorzugsweise Palladium auf Aluminiumoxid eingesetzt. Gute Resultate wurden mit relativ niedrigen Palladiumgehalten von beispielsweise 1% erzielt.

Der Wasserstoffdruck bei der Hydrierung ist vorzugsweise grösser als 10 bar und liegt beispielsweise bei ca. 50 bar. Als Reaktionstemperatur wird vorzugsweise eine Temperatur von 150 bis 220 °C eingestellt, beispielsweise 180 °C.

Die Hydrierung wird vorzugsweise kontinuierlich durchgeführt. Hierzu wird das Dinitril zusammen mit Wasserstoffin einem geeigneten Reaktor über den Katalysator geleitet.

Als Kohlenstoffkette A kommen vorzugsweise 1,2-Ethandiyl, 1,3-Propandiyl, 1,4-Butandiyl oder 1,3-Butandiyl (≡ 1-Methyl-1,3-propandiyl) in Frage, entsprechend den Dinitrilen Bernsteinsäuredinitril, Glutarsäuredinitril, Adipinsäuredinitril oder 2-Methylglutarsäuredinitril.

Es ist möglich, dem Dinitril II bis zur doppelten molaren Menge des entsprechenden cyclischen Amins 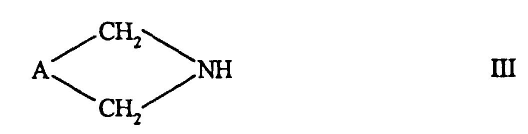 worin A die oben genannte Bedeutung hat,
zuzusetzen, d. h. bis zu einem molaren Verhältnis II:III = 1:2, wobei das Amin III in das Produkt I eingebaut wird. Dies hat den Vorteil, dass auf diese Weise das im erfindungsgemässen Verfahren auch als Nebenprodukt entstehende cyclische Amin III recycliert und letztlich in das gewünschte Produkt I übergeführt werden kann.

Vorzugsweise wird als Dinitril II 2-Methylglutarsäuredinitril und als cyclisches Amin m 3-Methylpiperidin eingesetzt.

Besonders bevorzugt als Ausgangsmaterial ist das 2-Methyl-glutarsäuredinitril, welches als neues Produkt das 1,5-Bis(3-methylpiperidino)-2-methylpentan der Formel 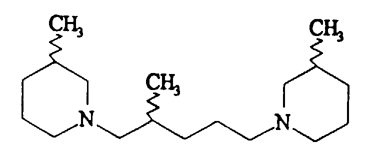 liefert. Diese Verbindung eignet sich insbesondere als Katalysator zur Herstellung von Polyurethanen, Polyurethan / Polyharnstoff-Gemischen und Polyharnstoffen. Die Polymere können damit sowohl als Elastomere als auch in Form von Schäumen hergestellt werden.

Die folgenden Beispiele verdeutlichen die Durchführung des erfindungsgemässen Verfahrens.

Die Wasserstoffströme sind auf Normalbedingungen bezogen, der Durchsatz ist jeweils in g Edukt pro g Katalysator und Stunde angegeben. Die Produktzusammensetzung wurde jeweils gaschromatographisch bestimmt.

### Beispiele:

### Beispiel 1:

In einen Reaktor (13 mm Ø) wurden 3 g eines Pd/Al₂O₃-Katalysators (1% Pd, Korngrösse 0,315 - 1 mm) eingefüllt. Im Wasserstoffstrom (120 ml/min) wurde eine Temperatur von 180 °C und ein Druck von 50 bar eingestellt. Dann wurde 99,8%-iges 2-Methylglutarsäuredinitril (MGN) zudosiert. Der Durchsatz betrug 2,1 g/(g·h), das molare Verhältnis MGN:H₂ 1: 5. Bei vollständigem Umsatz stellten sich im Produktstrom nach 40 h Reaktionszeit folgende Konzentrationen ein (GC): 82,5% 1,5-Bis(3-methylpiperidino)-2-methylpentan, 0,4% Isomerengemisch aus 2-Methyl-5-(3-methylpiperidino)pentannitril (Hauptkomponente) und 4-Methyl-5-(3-methylpiperidino)pentannitril, 8,4% 3-Methylpiperidin, Rest: 8,7%. Dieses Produktgemisch war wasserklar.

### Beispiel 2:

In einen Reaktor (13 mm Ø) wurden 3 g eines Pd/MgCl₂/Al₂O₃-Katalysators (Korngrösse 0,315 - 1 mm) eingefüllt, der 1% Pd und 1,2% Mg enthielt. Im Wasserstoffstrom (120 ml/min) wurde eine Temperatur von 170 °C und ein Druck von 50 bar eingestellt. Dann wurde 99,8%-iges 2-Methylglutarsäuredinitril (MGN) zudosiert. Der Durchsatz betrug 2,1 g/(g·h). Bei vollständigem Umsatz stellten sich im Produktstrom nach 22 h Reaktionszeit folgende Konzentrationen ein (GC): 78,4% 1,5-Bis(3-methylpiperidino)-2-methylpentan, 8,3% Isomerengemisch aus 2-Methyl-5-(3-methylpiperidino)pentannitril (Hauptkomponente) und 4-Methyl-5-(3-methylpiperidino)pentannitril, 3,0% 3-Methylpiperidin, Rest: 10,3%. Das Produktgemisch war wasserklar.

### Beispiel 3:

In einen Reaktor (13 mm Ø) wurden 3 g eines Pd/MgCl₂/Al₂O₃-Katalysators (Korngrösse 0,315 - 1 mm) eingefüllt, der 1% Pd und 1,2% Mg enthielt. Im Wasserstoffstrom (120 ml/min) wurde eine Temperatur von 190 °C und ein Druck von 50 bar eingestellt. Dann wurde 99,8%-iges 2-Methylglutarsäuredinitril (MGN) zudosiert. Der Durchsatz betrug 2,1 g/(g·h). Bei vollständigem Umsatz stellten sich im Produktstrom folgende Konzentrationen ein (GC): 76,3% 1,5-Bis(3-methylpiperidino)-2-methylpentan, 4,3% 2-Methyl-5-(3-methylpiperidino)pentannitril, 15,3% 3-Methylpiperidin, Rest: 4,1% (Reaktionszeit: 90 h). Das wasserklare Produkt wurde destilliert und 1,5-Bis(3-methylpiperidino)-2-methylpentan in einer Reinheit von 99,5% erhalten (108 °C/1,3 mbar).

Analytische Daten:
1,5-Bis(3-methylpiperidino)-2-methylpentan:

| | | | |
|---|---|---|---|
| C₁₈H₃₆N₂Berechnet | C 77,1 | H 12,9 | N 10,0 |
| Gefunden | C 77,2 | H 13,4 | N 10,0 |
| Molmasse | 280 (MS) | | |

- ¹H-NMR (CDCl₃, 400 MHz) δ: (Diastereomerengemisch): 2,65 - 2,90 (m, 4H, Ring-N―CH₂);
2,25 (t, 2 H, N―C*H*₂―CH₂);
2,02 (m, 2 H, N―C*H*₂―CH);
1,34 - 1,82 (m, 16 H);
0,78 - 1,08 (m, 12 H, CH₃ u. a.).
- ¹³C-NMR (CDCl₃, 100 MHz) δ: (Diastereomerengemisch): 54,1 - 66,3 (N-CH₂);
33,3 (N―CH₂―CH₂―*C*H₂);
30,5 - 31,2 (CH);
24,5 - 25,7 (N―CH₂―*C*H₂);
18,5 - 19,9 (CH₃).

2-Methyl-5-(3-methylpiperidino)pentannitril/ 4-Methyl-5-(3-methylpiperidino)pentannitril (Isomerengemisch, ca. 85 : 15)

| | | | |
|---|---|---|---|
| C₁₂H₂₂N₂Berechnet | C 74,2 | H 11,4 | N 14,4 |
| Gefunden | C 74,2 | H 11,6 | N 14,9 |

- ¹H-NMR (CDCl₃, 400 MHz) δ: (Hauptkomponente): 1,40 - 2,85 (m, 16 H, CH + CH₂);
1,32 (d, 3 H, C*H*₃―CH―CN);
0,86 (d, 3 H, Ring-CH₃).
- ¹³C-NMR (CDCl₃, 100 MHz) δ: (Hauptkomponente): 123,01 (s); 62,20 (t); 58,28 (t); 54,12 (t);
33,13 (t); 32,23 (t); 31,19 (d); 25,62 (t);
25,47 (d); 24,41 (d); 19,77 (q); 18,07 (q).

### Beispiele 4-7:

Einsatz von 1,5-Bis(3-methylpiperidino)-2-methylpentan als Polyurethan-Katalysator:

### Abkürzungen:

VL: Desmodur® VL von Bayer, aromatisches Diisocyanat mit ca. 32% -NCO
D550U: Desmophen® 550U, Polypropylenglykol von Bayer, trifunktionell mit 10,5% ―OH
DBU: Diazabicyclo[5.4.0]undec-7-en
BMPMP: 1,5-Bis(3-methylpiperidino)-2-methylpentan

Als Vergleichskatalysator diente DBU. Desmophen wurde mit dem Amin (DBU oder BMPMP) vorgelegt und gut gemischt. Es entstand eine Lösung. In Beispiel 6 und 7 wurde zusätzlich Wasser zugegeben. Es entstand eine Emulsion / Lösung. Eine eingewogene Menge Isocyanat (VL) wurde zum Zeitpunkt t = 0 unter heftigem Rühren zugesetzt. Es wurden die Zeitpunkte notiert zu denen
- die Lösung nicht mehr trübe ist,
- eine deutliche Erwärmung feststellbar ist,
- die Mischung fest wird.

Die Resultate (BMPMP im Vergleich zu DBU) gehen aus der Tabelle 1 hervor.

Die Zugabe von Wasser in den Beispielen 6 und 7 bewirkt eine teilweise Hydrolyse des Isocyanats zum entsprechenden Amin und die Freisetzung von CO₂. Das entsprechende Amin reagiert mit dem verbleibenden Isocyanat zu einer Harnstoffverbindung, während das CO₂ als Treibmittel bei der Schaumbildung wirkt.

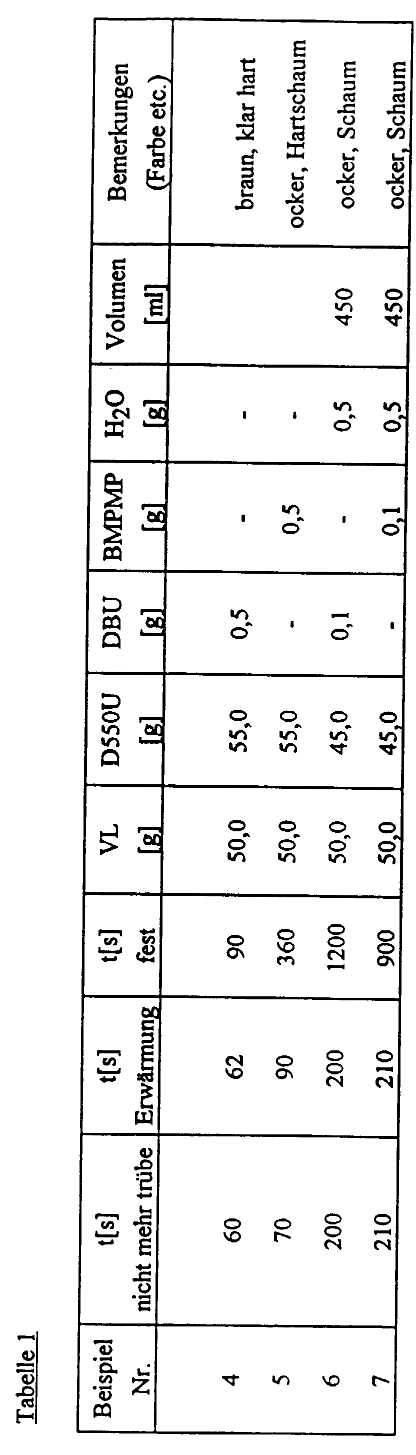

### Beispiel 8:

### Untersuchung zum Reaktionsmechanismus:

In einen Reaktor (13 mm Ø) wurden 3 g eines Pd/Al₂O₃ Katalysators (1% Pd, Korngrösse 0,315 - 1 mm) eingefüllt. Im Wasserstoffstrom (120 ml/min) wurde eine Temperatur von 180 °C und ein Druck von 50 bar eingestellt. Dann wurde 99,8%-iges 2-Methylglutarsäuredinitril (MGN) zudosiert. Der Durchsatz betrug 2,1 g/(g·h). Bei vollständigem Umsatz stellten sich im Produktstrom nach 6 h Reaktionszeit folgende Konzentrationen ein (GC): 78% 1,5-Bis(3-methylpiperidino-2-methylpentan, 21% 3-Methylpiperidin (MPI), Rest: 1%. Anschliessend wurde statt 2-Methylglutarsäuredinitril (MGN) 3-Methylpiperidin (MPI) über den Katalysator geleitet. MPI passierte zu >99,5% den Katalysator unverändert. Anschliessend wurde MPI und MGN im molaren Verhältnis 2 : 1 über den Katalysator geleitet. Im Produktstrom stellten sich folgende Konzentrationen ein (GC) : 18,9% MPI, 70,6%1,5-Bis(3-methylpiperidino)-2-methylpentan, Rest: 10,5%. Die Versuchsergebnisse zeigen, dass MPI allein nicht zu 1,5-Bis(3-methylpiperidino)-2-methylpentan umgesetzt wird. MPI wird aber, wenn es dem MGN zugesetzt wird, in das erwünschte Produkt eingebaut.

### Beispiel 9

In einen Reaktor (13 mm Ø) wurden 3 g eines Pd/Al₂O₃ Katalysators (1% Pd, Korngrösse 0,315 - 1 mm) eingefüllt. Im Wasserstoffstrom (140 ml/min) wurde eine Temperatur von 180 °C und ein Druck von 50 bar eingestellt. Dann wurde Glutarsäuredinitril zudosiert. Der Durchsatz betrug 2,1 g/(g·h). Bei einem Umsatz von 98,6% enthielt der Produktstrom nach 4 h Reaktionszeit 34,6% 5-Piperidinopentannitril, 32,4% 1,5-Dipiperidinopentan und 5,5% Piperidin (GC).

### Beispiel 10:

In einen Reaktor (13 mm Ø) wurden 3 g eines Pd/Al₂O₃ Katalysators (1% Pd, Korngrösse 0,315 - 1 mm) eingefüllt. Im Wasserstoffstrom (120 ml/min) wurde eine Temperatur von 180 °C und ein Druck von 50 bar eingestellt. Dann wurde Adipodinitril zudosiert. Der Durchsatz betrug 2,1 g/(g·h). Bei einem Umsatz von 98,8% enthielt der Produktstrom nach 3 h Reaktionszeit 26% 6-(Hexahydro-1*H*-azepin-1-yl)hexannitril, 20% 1,6-Bis(hexahydro1*H*-azepin-1-yl)hexan und 13% Hexahydro-1*H*-azepin (GC).

### Beispiele 11 - 13:

In einem Reaktor (13 mm Ø) wurden 3 g eines Pd/Al₂O₃ -Katalysators (1% Pd, Korngrösse 0,315 - 1.0 mm) eingefüllt. Der Reaktor wurde im Wasserstoffstrom (120 ml/min) bei 50 bar auf 180 °C aufgeheizt. Dann wrude mit der Zudosierung von 99,8%igem 2-Methylglutarsäuredinitril begonnen. Das molare Verhältnis MGN:H₂ und der MGN-Durchsatz wurden variiert und folgende Resultate erhalten.

### Beispiel 11:

MGN:H₂ = 1:10
Durchsatz: 2,10 g/(g·h)

| Produktzusammensetzung | |
|---|---|
| 1,5-Bis(3-methylpiperidino)-2-methylpentan | 71,2% |
| 2(4)-Methyl-5-(3-methylpiperidino)pentannitril | 0,7% |
| 2-Methylglutarsäuredinitril | 0% |
| 3-Methylpiperidin | 9,2% |
| Rest | 18,9% |

### Beispiel 12:

MGN:H₂ = 1:3,4
Durchsatz: 2,10 g/(g·h)

| Produktzusammensetzung | |
|---|---|
| 1,5-Bis(3-methylpiperidino)-2-methylpentan | 44,2% |
| 2(4)-Methyl-5-(3-methylpiperidino)pentannitril | 20,1% |
| 2-Methylglutarsäuredinitril | 8,4% |
| 3-Methylpiperidin | 3,6% |
| Rest | 23,7% |

### Beispiel 13:

MGN:H₂ = 1:5
Durchsatz: 1,73 g/(g·h)

| Produktzusammensetzung | |
|---|---|
| 1,5-Bis(3-methylpiperidino)-2-methylpentan | 80,4% |
| 2(4)-Methyl-5-(3-methylpiperidino)pentannitril | 3,6% |
| 2-Methylglutarsäuredinitril | 0% |
| 3-Methylpiperidin | 5,7% |
| Rest | 10,2% |

Die Beispiele 1 und 11 - 13 zeigen, dass die höchste Selektivität für die Bildung der Bis(methylpiperidino)-Verbindung bei einem molaren Verhältnis MGN:H₂ von ungefähr 1:5 erreicht wird.

## Patentansprüche

1. Verfahren zur Herstellung von tertiären Diaminen der allgemeinen Formel 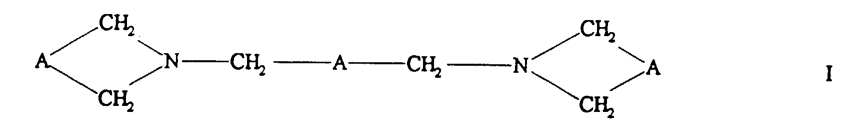 worin A jeweils für eine gegebenenfalls mit einer oder mehreren C₁-C₄-Alkylgruppen substituierte zwei- bis viergliedrige aliphatische Kohlenstoffkette steht, dadurch gekennzeichnet, dass ein Dinitril der allgemeinen Formel
N≡C―A―C≡N II
worin A die oben genannte Bedeutung hat, bei einer Reaktionstemperatur von 100 - 250 °C unter erhöhtem Druck in Gegenwart eines Palladium-Trägerkatalysators mit Wasserstoff umgesetzt wird.

2. Verfahren nach Anspruch 1, dadurch gekennzeichnet, dass als Palladium-Trägerkatalysator Palladium auf Aluminiumoxid eingesetzt wird.

3. Verfahren nach Anspruch 1 oder 2, dadurch gekennzeichnet, dass der Wasserstoffdruck grösser als 10 bar ist.

4. Verfahren nach einem oder mehreren der Ansprüche 1 bis 3, dadurch gekennzeichnet, dass die Reaktionstemperatur 150 - 220 °C beträgt.

5. Verfahren nach einem oder mehreren der Ansprüche 1 bis 4, dadurch gekennzeichnet, dass die Umsetzung mit Wasserstoff kontinuierlich erfolgt.

6. Verfahren nach einem oder mehreren der Ansprüche 1 bis 5, dadurch gekennzeichnet, dass als Dinitril (II) Bernsteinsäuredinitril, Glutarsäuredinitril, Adipinsäuredinitril oder 2-Methylglutarsäuredinitril eingesetzt wird.

7. Verfahren nach einem oder mehreren der Ansprüche 1 bis 6, dadurch gekennzeichnet, dass dem Dinitril II das entsprechende cyclische Amin der allgemeinen Formel 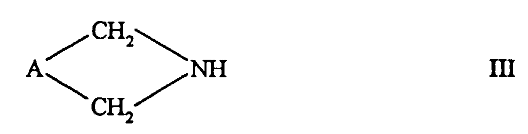 worin A die in Anspruch 1 genannte Bedeutung hat in einer Menge von bis zu 2 mol auf 1 mol Dinitril zugesetzt wird.

8. Verfahren nach Ansprüchen 6 und 7, dadurch gekennzeichnet, dass als Dinitril II 2-Methylglutarsäuredinitril und als cyclisches Amin III 3-Methylpiperidin eingesetzt wird.

9. 1,5-Bis(3-methylpiperidino)-2-methylpentan der Formel

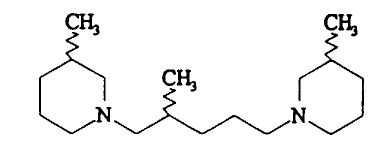

10. Verwendung von 1,5-Bis(3-methylpiperidino)-2-methylpentan der Formel 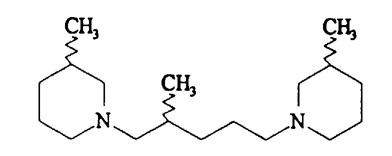 als Katalysator zur Herstellung von Polyurethanen, Polyurethan / Polyharnstoff-Gemischen oder Polyharnstoffen in Elastomer- und / oder Schaumform.

## Claims

1. A method for producing tertiary diamines having the general formula 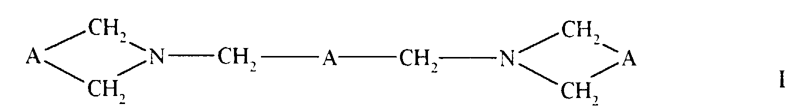 wherein A represents a respective two- to four-unit aliphatic carbon chain optionally substituted with one or several C₁-C₄ alkyl groups, characterised in that a dinitrile having the general formula
N≡C―A―C≡N II
wherein A has the above specified meaning is reacted with hydrogen at a reaction temperature of 100 - 250°C under elevated pressure in the presence of a supported palladium catalyst.

2. The method according to claim 1,characterised in that palladium on aluminum oxide is used as said supported palladium catalyst.

3. The method according to claim 1 or 2, characterised in that the hydrogen pressure is higher than 10 bar.

4. The method according to one or several of claims 1 to 3, characterised in that the reaction temperature is 150 - 220°C.

5. The method according to one or several of claims 1 to 4, characterised in that reaction with hydrogen is performed continuously

6. The method according to one or several of claims 1 to 5, characterised in that said dinitrile (II) used is succinic dinitrile, glutaric dinitrile, adipic dinitrile or 2-methylglutaric dinitrile

7. The method according to one or several of claims 1 to 6, characterised in that the corresponding cyclic amine having the general formula 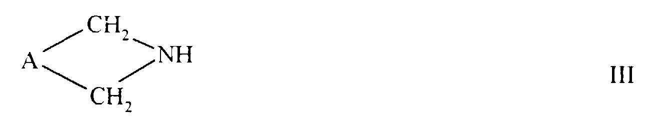 wherein A has the meaning specified in claim 1, is added to said dinitrile II in a quantity of up to 2 mol for 1 mol dinitrile.

8. The method according to claims 6 and 7, characterised in that said dinitrile II used is 2-methylglutaric dinitrile, and the cyclic amine III used is 3-methylpiperidine.

9. 1,5-bis(3-methylpiperidino)-2-methylpentane having the formula

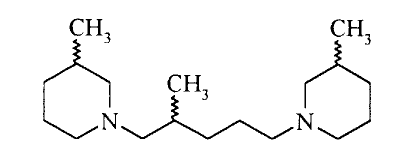

10. Use of 1,5-bis(3-methylpiperidino)-2-methylpentane having the formula 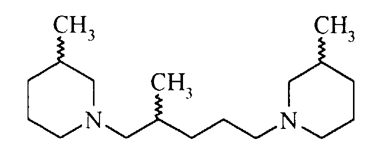 as a catalyst for producing polyurethanes, polyurethane/polyurea mixtures or polyurea in elastomer and/or foam form.

## Revendications

1. Procédé pour la préparation de diamines tertiaires de la formule générale 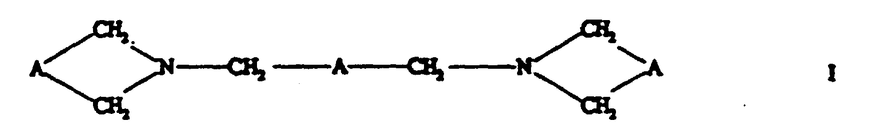 dans laquelle A représente une chaîne de carbone aliphatique à 2 jusqu'à 4 éléments, éventuellement substituée par un ou plusieurs groupes alkyle C₁-C₄, caractérisé en ce qu'on met en réaction un dinitrile de la formule générale
N≡C―A―C≡N II
dans laquelle A a la signification précitée, à une température réactionnelle de 100 - 250°C sous pression élevée en présence d'un catalyseur porteur de palladium avec de l'hydrogène.

2. Procédé selon la revendication 1, caractérisé en ce qu'en tant que catalyseur porteur de palladium, on utilise du palladium sur de l'oxyde d'aluminium.

3. Procédé selon la revendication 1 ou 2, caractérisé en ce que la pression d'hydrogène est supérieure à 10 bars.

4. Procédé selon une ou plusieurs des revendications 1 à 3, caractérisé en ce que la température réactionnelle est de 150 - 220°C.

5. Procédé selon une ou plusieurs des revendications 1 à 4, caractérisé en ce que la réaction s'effectue en continu avec de l'hydrogène.

6. Procédé selon une ou plusieurs des revendications 1 à 5, caractérisé en ce qu'en tant que dinitrile (II), on utilise le dinitrile de l'acide succinique, le dinitrile de l'acide glutarique, le dinitrile de l'acide adipinique ou le dinitrile de l'acide 2-méthylglutarique.

7. Procédé selon une ou plusieurs des revendications 1 à 6, caractérisé en ce qu'au dinitrile (Il), on ajoute l'amine cyclique correspondante de la formule générale 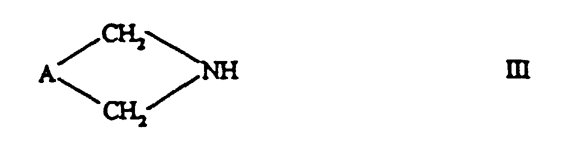 dans laquelle A a la signification citée dans la revendication 1 dans une quantité de 2 à 1 mole de dinitrile.

8. Procédé selon les revendications 6 et 7, caractérisé en ce qu'en tant que dinitrile II, on utilise le dinitrile de l'acide 2-méthylglutarique et en tant qu'amine III cyclique, on utilise la 3-méthylpipéridine.

9. 1,5-bis(3-méthylpipéridino)-2-méthylpentane de la formule

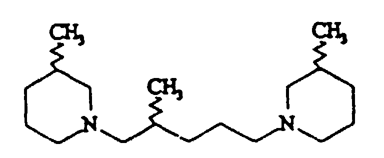

10. Utilisation de 1,5-bis(3-méthylpipéridino)-2-méthylpentane de la formule 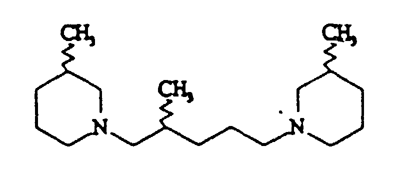 en tant que catalyseur pour la préparation de polyuréthannes, mélanges polyuréthanne/polyurée dans la forme élastomère et/ou alvéolaire.
